(19) Europäisches Patentamt

European Patent Office

Office européen des brevets

(11) Veröffentlichungsnummer: **0 281 977 B1**

# (12) EUROPÄISCHE PATENTSCHRIFT

(45) Veröffentlichungstag der Patentschrift: **06.05.92**

(21) Anmeldenummer: **88103457.3**

(22) Anmeldetag: **05.03.88**

(51) Int. Cl.⁵: **C07C 17/38**, B04C 5/081

(54) **Verfahren und Vorrichtung zur Abscheidung von festen Partikeln aus flüssigen, chlorierten Kohlenwasserstoffen.**

(30) Priorität: **12.03.87 DE 3708010**

(43) Veröffentlichungstag der Anmeldung:
**14.09.88 Patentblatt 88/37**

(45) Bekanntmachung des Hinweises auf die
Patenterteilung:
**06.05.92 Patentblatt 92/19**

(84) Benannte Vertragsstaaten:
**BE DE FR GB IT NL**

(56) Entgegenhaltungen:
**DE-A- 3 219 352**

(73) Patentinhaber: **HOECHST AKTIENGESELL-
SCHAFT
Postfach 80 03 20
W-6230 Frankfurt am Main 80(DE)**

(72) Erfinder: **Bennoit, Horst
Klausenerstrasse 90,
W-6620 Völklingen(DE)**
Erfinder: **Fröhlich, Walter, Dr.
Kampenwandstrasse 11
W-8269 Burgkirchen(DE)**
Erfinder: **Höltermann, Rolf
Hochgernstrasse 21
W-8265 Neuötting(DE)**
Erfinder: **Krumböck, Reinhard
Lohnerstrasse 40
W-8269 Burgkirchen(DE)**

## Beschreibung

Die Erfindung betrifft ein Verfahren und eine Vorrichtung zur Abscheidung von festen Partikeln aus einem Gemisch mit mindestens einem flüssigen, chlorierten Kohlenwasserstoff gemäß Anspruch 1.

Bei der Herstellung von chlorierten Kohlenwasserstoffen, insbesondere, wenn letztere unter erhöhtem Druck verdampft werden, wie auch bei der thermischen Spaltung verdampfter, chlorierter Kohlenwasserstoffe und deren nachfolgender Abkühlung, entstehen durch Nebenreaktionen unerwünschte, meist stark kohlenstoffhaltige Feststoffe. Sie sind häufig in flüssigen, chlorierten Kohlenwasserstoffen oder deren Gemischen enthalten, die beispielsweise bei der Verdampfung zurückbleiben oder durch Kondensation entstehen. Diese Feststoffe führen zu Produktionsstörungen, beispielsweise durch Verstopfung oder Querschnittsverengung sowie auch durch schlechten Wärmeübergang. In solchen Fällen muß die Anlage abgestellt und gereinigt werden, was zu empfindlichen Produktionsausfällen sowie zu unerwünschten Emissionen führt. Es besteht daher die Aufgabe, solche flüssigen, chlorierten Kohlenwasserstoffe oder deren Gemische von festen Partikeln möglichst weitgehend zu befreien, wobei möglichst wenig Emissionen erfolgen sollen.

Hierzu ist es bekannt, beispielsweise Siebkorbfilter zu verwenden, die jedoch häufig, umständlich und mit beträchtlichen Emissionen gereinigt werden müssen, da sie schnell verstopfen. Eine Verbesserung bringt das in DE-PS 32 19 352 beschriebene Verfahren, bei dem anstelle des Siebkorbfilters ein Spaltfilter mit nachgeschaltetem Abscheider eingesetzt wird. Nachteilig bleibt weiterhin der relativ hohe Druckverlust im Filter sowie eine gewisse Störanfälligkeit, unter anderem wegen mechanisch bewegter Apparateteile, mit der Notwendigkeit, zur Beseitigung der Störung die Apparate öffnen zu müssen, wobei Emissionen auftreten. Solche Emissionen können auch bei den vergleichsweise hohen Drucken im Filter an der Durchführung der Welle durch das Apparategehäuse entstehen. Ferner kann es in Siebkorbfiltern zu Feststoff-Agglomerationen kommen, die die Verstopfungsgefahr erhöhen.

Es wurde nun ein Verfahren gefunden, das die oben beschriebenen Nachteile nicht aufweist. Das neue Verfahren zur Abscheidung von festen Partikeln aus einem Gemisch mit mindestens einem flüssigen, chlorierten Kohlenwasserstoff ist dadurch gekennzeichnet, daß das nicht mehr als 2 Gew.-%, bezogen auf das Gemisch, Feststoffe enthaltende Flüssigkeitsgemisch in mindestens einen Hydrozyklon mit nachfolgendem Abscheider eingeleitet wird, wobei es im Hydrozyklon in einem Kreis mit einem maximalen Radius von 1 bis 50 cm strömt, der Quotient aus dem Quadrat der Eingangs-Strömungsgeschwindigkeit des feststoffhaltigen Flüssigkeitsgemisches und dem maximalen Kreisradius, mit dem das feststoffhaltige Flüssigkeitsgemisch im Hydrozyklon strömt, von 50 bis 2 000 $m/s^2$ beträgt und das Verhältnis des Abstandes zwischen dem Eintrag des feststoffhaltigen Flüssigkeitsgemisches im oberen Teil des Hydrozyklons und dem Austrag des an Feststoff angereicherten Flüssigkeitsgemisches am unteren Ende des Hydrozyklons zum maximalen Kreisradius des im Hydrozyklon strömenden feststoffhaltigen Flüssigkeitsgemisches 3 bis 30 beträgt.

Das feststoffhaltige Flüssigkeitsgemisch soll nicht mehr als 2 Gew.-%, bezogen auf dieses Gemisch, Feststoffe enthalten, da bei höheren Feststoffgehalten das erfindungsgemäße Verfahren im allgemeinen nicht mehr störungsfrei durchgeführt werden kann. Nach unten ist der Feststoffgehalt der Flüssigkeit nicht begrenzt, im allgemeinen wird man einen Feststoffgehalt von 0,0001 Gew.-%, bezogen auf das feststoffhaltige Flüssigkeitsgemisch, nicht unterschreiten, da bei so niedrigen Gehalten das erfindungsgemäße Verfahren gegenüber anderen bekannten Verfahren (zum Beispiel Filter) keine wesentlichen Vorteile mehr aufweist. Vorzugsweise enthält das feststoffhaltige Flüssigkeitsgemisch 0,0005 bis 0,5 Gew.-%, bezogen auf dieses Gemisch, Feststoffe.

Die Korngröße des Feststoffes kann in weiten Grenzen schwanken. Vorteilhaft sollte der Feststoff eine gewichtsmittlere Teilchengröße von 50 bis 2 000 μm aufweisen. Für Feststoffe mit über 2 000 μm mittlerer Teilchengröße ist das erfindungsgemäße Verfahren zwar anwendbar, jedoch können hier auch andere bekannte Verfahren, zum Beispiel die Abtrennung mit einem grobmaschigen Sieb, angewendet werden. Unterhalb 50 μm gewichtsmittlerer Teilchengröße ist das erfindungsgemäße Verfahren ebenfalls anwendbar, jedoch besteht im allgemeinen zur Abtrennung von Feststoffen mit einer derart geringen Teilchengröße häufig keine Notwendigkeit. Besonders gute Ergebnisse werden erhalten, wenn ein Gemisch eingesetzt wird, dessen feste Partikel eine gewichtsmittlere Teilchengröße von 150 bis 1 000 μm aufweisen.

Das erfindungsgemäße Verfahren kann in einem weiten Temperaturbereich durchgeführt werden, der unter anderem von den gewählten Verfahrensparametern abhängt, die beispielsweise zur Verdampfung beziehungsweise zur thermischen Spaltung oder auch Kondensation der chlorierten Kohlenwasserstoffe angewendet werden. Vorzugsweise wird bei 10 bis 270 °C und insbesondere bei 50 bis 160 °C gearbeitet.

Für den Druck gilt sinngemäß das gleiche, wie im vorangehenden Absatz über die Temperatur gesagt. Aus rein wirtschaftlichen Gründen wird man sehr hohe Drucke vermeiden, da hierfür unnötig teure Apparaturen verwendet werden müßten. Vorzugsweise wird das neue Verfahren bei einem Druck von 1 bis 40 bar und insbesondere bei 8 bis 25 bar durchgeführt.

Die festen Partikel können im Gemisch mit einem oder mehreren flüssigen, chlorierten Kohlenwasserstoffen vorliegen. "Flüssig" heißt, unter den gewählten Betriebsbedingungen von Temperatur und Druck flüssig. Die chlorierten Kohlenwasserstoffe können gesättigt, ungesättigt, verzweigt und zyklisch sein, sie können von 1 bis 6 Kohlenstoffatome und von 1 bis zu 5 Chloratome enthalten. Bevorzugt wird das erfindungsgemäße Verfahren für feststoffhaltige Flüssigkeitsgemische verwendet, die einen überwiegenden Anteil von mindestens einem chlorierten Kohlenwasserstoff mit 2 C-Atomen enthalten. Wegen der besonderen technischen Bedeutung wird das erfindungsgemäße Verfahren insbesondere angewendet für solche feststoffhaltigen Flüssigkeitsgemische, die aus mindestens 25 Gew.-%, bezogen auf das Gemisch, 1,2-Dichlorethan bestehen. Das Flüssigkeitsgemisch kann neben den chlorierten Kohlenwasserstoffen noch geringe Mengen, im allgemeinen nicht über 1 Gew.-%, bezogen auf das Gemisch, von anderen organischen Verbindungen enthalten, beispielsweise nichtchlorierte Kohlenwasserstoffe und Verbindungen, die neben Kohlenstoff, Wasserstoff und Chlor noch Sauerstoffatome enthalten.

Erfindungsgemäß wird das feststoffhaltige Flüssigkeitsgemisch in mindestens einen Hydrozyklon mit nachfolgendem Abscheider eingeleitet. Der Hydrozyklon kann verschiedener Bauart sein. Er kann beispielsweise unten konisch zulaufend oder auch zylindrisch ausgeführt sein. Vorteilhaft sollte das feststoffhaltige Flüssigkeitsgemisch im Hydrozyklon in einem Kreis mit einem maximalen Radius von 1 bis 50 cm strömen. Es sind zwar auch darüber- und darunterliegende Kreisströmungsradien möglich, doch werden mit diesen im allgemeinen nicht so gute Ergebnisse erzielt. Insbesondere sollte das feststoffhaltige Flüssigkeitsgemisch in einem Kreis mit einem maximalen Radius von 5 bis 30 cm strömen.

Es hat sich für die Zwecke der Erfindung als günstig erwiesen, wenn die Betriebsparameter so gewählt werden, daß der Quotient aus dem Quadrat der Geschwindigkeit, mit dem das feststoffhaltige Flüssigkeitsgemisch in den Hydrozyklon einströmt, und dem maximalen Kreisradius, mit dem das feststoffhaltige Flüssigkeitsgemisch im Hydrozyklon strömt, von 50 bis 2 000 m/s$^2$ und insbesondere von 100 bis 1 500 m/s$^2$ beträgt.

Der Schlankheitsgrad des Hydrozyklons kann in beträchtlichen Grenzen schwanken, vorteilhaft beträgt das Verhältnis des Abstandes zwischen dem Eintrag des feststoffhaltigen Flüssigkeitsgemisches im oberen Teil des Hydrozyklons und dem Austrag des an Feststoff angereicherten Flüssigkeitsgemisches am unteren Ende des Hydrozyklons zum maximalen Kreisradius des im Hydrozyklon strömenden feststoffhaltigen Flüssigkeitsgemisches 3 bis 30 und insbesondere 5 bis 20.

Zur Verbesserung der Trennwirkung können mindestens 2 Hydrozyklone hintereinander geschaltet werden, wie es auch möglich ist, zur Bewältigung eines größeren Flüssigkeitsvolumens 2 oder mehrere Hydrozyklone parallel laufen zu lassen. In solchen Fällen ist es häufig nicht erforderlich, daß jeder Hydrozyklon nachfolgend einen eigenen Abscheider hat, sondern es können Abläufe mehrerer Hydrozyklone in einen Abscheider geführt werden.

Der Anteil des aus dem Hydrozyklon abgeführten feststoffreicheren Flüssigkeitsgemisches, bezogen auf das zugeführte feststoffhaltige Flüssigkeitsgemisch, ist vom Feststoffgehalt der zugeführten Flüssigkeit, der mittleren Teilchengröße des Feststoffs und dem Dichteunterschied zwischen den festen und flüssigen Bestandteilen abhängig. Im allgemeinen kann dieser Anteil kleiner gehalten werden bei größerer mittlerer Teilchengröße und größerem Dichteunterschied zwischen festen und flüssigen Bestandteilen. Häufig werden gute Ergebnisse erhalten, wenn der Anteil des an Feststoff angereicherten Flüssigkeitsgemisches 0,1 bis 10 Gew.-%, vorzugsweise 0,5 bis 5 Gew.-%, bezogen auf das dem Hydrozyklon zugeführte feststoffhaltige Flüssigkeitsgemisch, beträgt.

Aus dem Oberteil des Hydrozyklons wird die von Feststoffen weitgehend befreite Flüssigkeit abgeführt und nach bekannten Verfahren weiterverarbeitet. Sollte der Trenneffekt im ersten Hydrozyklon nicht ausreichend gewesen sein, kann hier ein weiterer Hydrozyklon eingeschaltet werden. Sofern nur noch geringe aber dennoch störende Mengen eines sehr feinkörnigen Feststoffes vorliegen, können diese auch über ein konventionelles Filter, das dann mit langen Standzeiten betrieben werden kann, abgetrennt werden.

Im unteren Teil des Zyklons wird das an Feststoffen angereicherte Flüssigkeitsgemisch ausgetragen und entweder kontinuierlich oder absatzweise einem nachgeschalteten Abscheider zugeführt. Bevorzugt wird das angereicherte Flüssigkeitsgemisch einer Sedimentation der festen Partikel, ohne Anwendung von Zentrifugalkräften, beispielsweise in einem Sedimentationsbehälter, unterworfen. Im unteren Teil des Sedimentationsbehälters wird der Feststoff, der noch etwas Flüssigkeit enthält, kontinuierlich oder absatzweise ausgetragen und gegebenenfalls nach Abtreibung flüchtiger Bestandteile einer Entsorgung, zum Beispiel Verbrennung, zugeführt.

Die im oberen Teil des Sedimentationsbehälters abgeführte, von Feststoffen weitgehend befreite Flüssigkeit, kann, je nach Qualität, entweder mit der oben aus dem Hydrozyklon abgeführten Flüssigkeit vereint, weiterverarbeitet oder in den Hydrozyklon zurückgeführt werden, auch eine weitere Behandlung in einem zweiten Hydrozyklon ist möglich. Als Sedimentationsbehälter sind zum Beispiel einfache Absetzbehälter vorteilhaft, solche mit konisch sich verjüngendem Boden sowie Eindicker geeignet.

In einer weiteren bevorzugten Ausführungsform des erfindungsgemäßen Verfahrens wird aus dem an Feststoffen angereicherten Flüssigkeitsgemisch, das aus dem Hydrozyklon abgeführt wird, mindestens ein Teil der flüssigen Bestandteile verdampft. In diesem Fall ist der dem Hydrozyklon nachfolgende Abscheider ein Verdampfer. Der aus diesem Abscheider entweichende Dampfstrom kann beispielsweise einer Destillationskolonne zugeführt werden.

Prinzipiell sind als Abscheider auch solche geeignet, die mit Zentrifugalkräften arbeiten, wie Dekanter oder (Schäl-)Zentrifugen. Jedoch sind diese in der Regel nicht erforderlich und wegen der hohen Kosten auch weniger vorteilhaft.

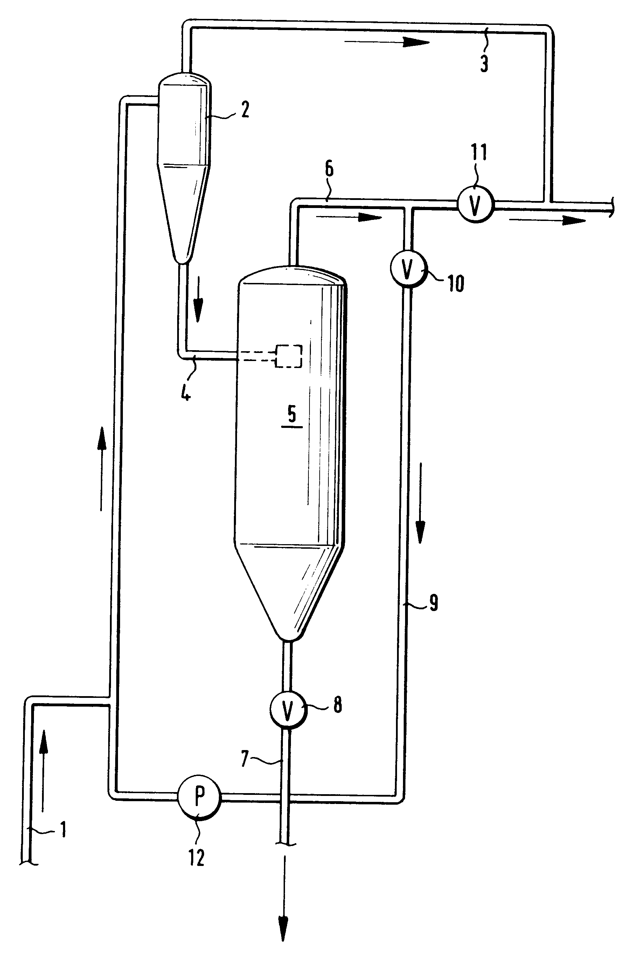

Gegenstand der Erfindung ist auch eine Vorrichtung zur Durchführung des weiter oben beschriebenen erfindungsgemäßen Verfahrens, wie sie beispielsweise in Figur 1 gezeigt ist und die besteht aus einem Hydrozyklon (2) mit einer Zuleitung (1), einer Ableitung im oberen Teil (3) und einer Ableitung im unteren Teil (4), die in ein senkrecht stehendes zylindrisches Gefäß (5) mit konisch sich verjüngendem Unterteil führt und dort in der Nähe der Zylinderachse, mindestens 20 % der inneren Gesamthöhe des Gefäßes (5) unterhalb des Oberteils dieses Gefäßes endet, einer Ableitung (6) im Oberteil des Gefäßes (5), die sich mit der Ableitung (3) aus dem Hydrozyklon (2) vereinigt und einer Ableitung (7) im unteren Teil des Gefäßes (5), die ein Absperrorgan (8) enthält.

Vorteilhaft enthält die erfindungsgemäße Vorrichtung zusätzlich eine Leitung (9) mit Absperrorgan (10) und Pumpe (12), die die Ableitung (6) mit der Zuleitung (1) verbindet und die zwischen dem Gefäß (5) und der Vereinigung mit der Ableitung (3) von der Ableitung (6) abzweigt sowie ein Absperrorgan (11) in der Ableitung (6) zwischen dem Abzweig der Leitung (9) und der Vereinigung mit der Ableitung (3). Das Gefäß (5) hat vorteilhaft ein Verhältnis der inneren Gesamthöhe zum inneren Durchmesser von 3 bis 10.

Das erfindungsgemäße Verfahren und die erfindungsgemäße Vorrichtung ermöglichen es, feste Partikel aus einem Gemisch mit mindestens einem flüssigen, chlorierten Kohlenwasserstoff so weit abzuscheiden, daß die gereinigte Flüssigkeit im weiteren Prozeß ohne Schwierigkeiten verwendet werden kann. Das Verfahren kann kontinuierlich in Vorrichtungen betrieben werden, die, mit Ausnahme der nicht in jedem Fall benötigten Pumpe (12), keine ständig mechanisch bewegten Teile enthält. Es werden lange Standzeiten bei einem Minimum an Emissionen erzielt.

Nachfolgende Beispiele sollen die Erfindung näher erläutern:

Beispiel 1

Es wird eine Apparatur gemäß Fig. 1 verwendet.

10 000 kg/h eines feststoffhaltigen Flüssigkeitsgemisches, das zu 96 Gew.-%, bezogen auf das gesamte Gemisch, aus einer Mischung von 1,2-Dichlorethan und Vinylchlorid besteht, und das 0,0018 Gew.-%, bezogen auf das gesamte Gemisch, Feststoffe einer gewichtsmittleren Teilchengröße von 810 $\mu$m enthält, werden über die Leitung (1) mit einer Temperatur von 80 °C unter einem Druck von 1,6 MPa mit einer Eingangs-Strömungsgeschwindigkeit von 4,6 m/s in den Oberteil eines Hydrozyklons (2) eingeleitet. Im Hydrozyklon (2) strömt das feststoffhaltige Flüssigkeitsgemisch in einem Kreis mit einem maximalen Radius von 5 cm, der Quotient aus dem Quadrat der Eingangs-Strömungsgeschwindigkeit und dem maximalen Kreisradius beträgt 423 m/s$^2$. Die Austragöffnung im unteren Teil des Hydrozyklons (2) befindet sich in 40 cm Entfernung von der Eintragsöffnung, das Verhältnis des Abstandes zwischen dem Eintrag und dem unteren Austrag zum maximalen Kreisradius im Hydrozyklon (2) beträgt 8. Aus der unteren Austragöffnung werden 250 kg/h eines an Feststoffen angereicherten Flüssigkeitsgemisches abgeführt, das 0,071 Gew.-%, bezogen auf das gesamte ausgetragene Gemisch, Feststoffe enthält, und über die Leitung (4) einem Sedimentationsbehälter (5) von 1 m$^3$ Inhalt zugeführt. Aus dem Hydrozyklon (4) werden also 2,5 Gew.-% des zugeführten, feststoffhaltigen Flüssigkeitsgemisches unten abgezogen.

Im Unterteil des Sedimentationsbehälters (5) werden 5 kg/h eines Feststoff-Flüssigkeitsgemisches über die Leitung (7) absatzweise in Portionen von je 40 kg abgezogen und einer Entsorgung zugeführt. Dieses Feststoff-Flüssigkeitsgemisch enthält 3,31 Gew.-% bezogen auf das Gemisch, trockenen Feststoff, das heißt 92 Gew.-% der Feststoffe, die mit dem feststoffhaltigen Flüssigkeitsgemisch in den Hydrozyklon (4) eingespeist werden, sind abgeschieden.

9 750 kg/h von Feststoffen weitgehend befreites Flüssigkeitsgemisch werden am Kopf des Hydrozyklons (2) über die Leitung (3) und 245 kg/h von Feststoffen weitgehend befreites Flüssigkeitsgemisch werden am Kopf des Sedimentationsbehälters (5) über die Leitung (6) abgezogen. Beide Flüssigkeitsströme werden vereinigt in einer Kolonne weiter destillativ aufgearbeitet. - Die Destillationskolonne läuft etwa 2,5mal länger als ohne die Feststoffabscheidung nach dem erfindungsgemäßen Verfahren.

Beispiel 2

Es wird eine Apparatur gemäß Fig. 1 verwendet.
17 500 kg/h eines feststoffhaltigen Flüssigkeitsmisches, das zu 97,5 Gew.-%, bezogen auf das gesamte Gemisch, aus einer Mischung von 1,2-Dichlorethan und Vinylchlorid besteht, und das 0,0025 Gew.-%, bezogen auf das gesamte Gemisch, Feststoffe einer gewichtsmittleren Teilchengröße von 970 $\mu$m enthält, werden über die Leitung (1) mit einer Temperatur von 95 °C unter einem Druck von 1,6 MPa mit einer Eingangs-Strömungsgeschwindigkeit von 8,3 m/s in den Oberteil eines Hydrozyklons (2) eingeleitet. Im Hydrozyklon (2) strömt das feststoffhaltige Flüssigkeitsgemisch in einem Kreis mit einem maximalen Radius von 5 cm, der Quotient aus dem Quadrat der Eingangs-Strömungsgeschwindigkeit und dem maximalen Kreisradius beträgt 1 380 m/s$^2$. Die Austragöffnung im unteren Teil des Hydrozyklons (2) befindet sich in 40 cm Entfernung von der Eintragsöffnung, das Verhältnis des Abstandes zwischen dem Eintrag und dem unteren Austrag zum maximalen Kreisradius im Hydrozyklon (2) beträgt 8. Aus der internen Austragöffnung werden 500 kg/h eines an Feststoffen angereicherten Flüssigkeitsgemisches abgeführt, das 0,092 Gew.-%, bezogen auf das gesamte ausgetragene Gemisch, Feststoffe enthält, und über die Leitung (4) einem Sedimentationsbehälter (5) von 1 m$^3$ Inhalt zugeführt. Aus dem Hydrozyklon (4) werden also 2,86 Gew.-% des zugeführten, feststoffhaltigen Flüssigkeitsgemisches unten abgezogen.

Im Unterteil des Sedimentationsbehälters (5) werden 15 kg/h eines breiigen Feststoff-Flüssigkeitsgemisches über die Leitung (7) kontinuierlich - absatzweise in Portionen von je 45 kg - abgezogen und einer Entsorgung zugeführt. Dieses Feststoff-Flüssigkeitsgemisch enthält 2,81 Gew.-% bezogen auf das Gemisch, trockenen Feststoff, das heißt 96,3 Gew.-% der Feststoffe, die mit dem feststoffhaltigen Flüssigkeitsgemisch über die Leitung (1) in den Hydrozyklon (4) eingespeist werden, sind abgeschieden.

17 485 kg/h von Feststoffen weitgehend befreites Flüssigkeitsgemisch werden am Kopf des Hydrozyklons (2) über die Leitung (3) und 485 kg/h von Feststoffen weitgehend befreites Flüssigkeitsgemisch werden am Kopf des Sedimentationsbehälters (5) über die Leitung (6) abgezogen. - Der über die Leitung (3) abgeführte Flüssigkeitsstrom wird in einer Kolonne weiter destillativ aufgearbeitet, der über die Leitung (6) abgeführte Flüssigkeitsstrom wird über die Leitung (9) und die Pumpe (12) in den Hydrozyklon (2) zurückgeführt. - Die Destillationskolonne läuft etwa 2,8mal länger als ohne die Feststoffabscheidung nach dem erfindungsgemäßen Verfahren.

**Patentansprüche**

1. Verfahren zur Abscheidung von festen Partikeln aus einem Gemisch mit mindestens einem flüssigen, chlorierten Kohlenwasserstoff, dadurch gekennzeichnet, daß das nicht mehr als 2 Gew.-% bezogen auf das Gemisch, Feststoffe enthaltende Flüssigkeitsgemisch in mindestens einen Hydrozyklon mit nachfolgendem Abscheider eingeleitet wird, wobei es im Hydrozyklon in einem Kreis mit einem maximalen Radius von 1 bis 50 cm strömt, der Quotient aus dem Quadrat der Eingangs-Strömungsgeschwindigkeit des feststoffhaltigen Flüssigkeitsgemisches und dem maximalen Kreisradius, mit dem das feststoffhaltige Flüssigkeitsgemisch im Hydrozyklon strömt, von 50 bis 2 000 m/s$^2$ beträgt und das Verhältnis des Abstandes zwischen dem Eintrag des feststoffhaltigen Flüssigkeitsgemisches im oberen Teil des Hydrozyklons und dem Austrag des an Feststoff angereicherten Flüssigkeitsgemisches am unteren Ende des Hydrozyklons zum maximalen Kreisradius des im Hydrozyklon strömenden feststoffhaltigen Flüssigkeitsgemisches 3 bis 30 beträgt.

2. Verfahren nach Anspruch 1, dadurch gekennzeichnet, daß das aus dem Hydroyzyklon ausgetragene, an Feststoff angereicherte Flüssigkeitsgemisch einer Sedimentation der festen Partikel ohne Anwendung von Zentrifugalkräften unterworfen wird.

3. Verfahren nach Anspruch 1 oder 2, dadurch gekennzeichnet, daß aus dem an Feststoffen angereicherten Flüssigkeitsgemisch, das aus dem Hydrozyklon abgeführt wird, mindestens ein Teil der flüssigen Bestandteile verdampft wird.

4. Verfahren nach einem oder mehreren der Ansprüche 1 bis 3, dadurch gekennzeichnet, daß das feststoffhaltige Flüssigkeitsgemisch überwiegend chlorierte Kohlenwasserstoffe mit 2 C-Atomen enthält.

5. Vorrichtung zur Durchführung des Verfahrens nach Anspruch 1, bestehend aus: einem Hydrozyklon (2) mit einer Zuleitung (1), einer Ableitung im oberen Teil (3) und einer Ableitung im unteren Teil (4), die in ein senkrecht stehendes zylindrisches Gefäß (5) mit konisch sich verjüngendem Unterteil führt und dort in der Nähe der Zylinderachse mindestens 20 % der inneren Gesamtbehälterhöhe des Gefäßes (5) unterhalb des Oberteils dieses Gefäßes endet, einer Ableitung (6) im Oberteil des Gefäßes (5), die sich mit der Ableitung (3) aus dem Hydrozyklon (2) vereinigt und einer Ableitung (7) im unteren Teil des Gefäßes (5), die ein Absperrorgan (8) enthält.

6. Vorrichtung nach Anspruch 5, die zusätzlich enthält: eine Leitung (9) mit Absperrorgan (10) und Pumpe (12), die die Ableitung (6) mit der Zuleitung (1) verbindet und die zwischen dem Gefäß (5) und der Vereinigung mit der Ableitung (3) von der Ableitung (6) abzweigt sowie ein Absperrorgan (11) in der Ableitung (6) zwischen dem Abzweig der Leitung (9) und der Vereinigung mit der Ableitung (3).

## Claims

1. A process for the removal of solid particles from a mixture containing at least one liquid chlorinated hydrocarbon, characterised in that the 2 % by weight, relative to the mixture, solids-containing liquid mixture is introduced in at least one hydrocyclone equipped with a downstream separator, the solids-containing liquid mixture flows in a circle having a maximum radius of from 1 to 50 cm, the quotient of the square of the inlet flow speed of the solids-containing liquid mixture to the maximum radius of the circle in which the solids-containing liquid mixture flows in the hydrocyclone is 50 to 2000 m/s$^2$ and the ratio of the distance between the inlet for the solids-containing liquid mixture in the upper part of the hydrocyclone and the outlet for the liquid mixture enriched in solids at the lower end of the hydrocyclone to the maximum radius of the circle in which the solids-containing liquid mixture flows in the hydrocyclone is 3 to 30.

2. The process as claimed in claim 1, characterised in that the liquid mixture which has been enriched in solids and is discharged from the hydrocyclone is subjected to sedimentation of the solid particles.

3. The process as claimed in claim 1 or 2, characterised in that at least a fraction of the liquid constituents are evaporated from the liquid mixture which has been enriched in solids and is drawn off from the hydrocyclone.

4. The process as claimed in either of claims 1 to 3, characterised in that the solids-containing liquid mixture contains predominantly chlorinated hydrocarbons having 2 carbon atoms.

5. Apparatus for carrying out the process as claimed in claim 1, which consists of: a hydrocyclone (2) having an inlet (1), an outlet in the upper part (3) and an outlet in the lower part (4) which leads into a vertical cylindrical vessel (5) having a conically tapering lower section, and which ends there, near to the cylinder axis at least 20 % of the total internal height of the vessel (5) below the upper part of this vessel, an outlet (6) in the upper part of the vessel (5) which unites with the outlet (3) from the hydrocyclone (2), and an outlet (7) in the lower section of the vessel (5) which contains a shut-off device (8).

6. Apparatus as claimed in claim 5, which additionally contains: a line (9) which has a shut-off device (10) and a pump (12) and which connects the outlet (6) to the inlet (1) and which branches off from the outlet (6) between the vessel (5) and the junction with the outlet (3), and also a shut-off device (11) in the outlet (6) between the point where the line (9) branches off and the junction with the outlet (3).

## Revendications

1. Procédé pour séparer des particules solides d'un mélange comportant au moins un hydrocarbure liquide chloré, procédé caractérisé en ce qu'on introduit le mélange liquide, ne contenant pas plus de 2 % en poids, par rapport au mélange, de solides, dans au moins un hydrocyclone comportant un séparateur en aval, ce mélange s'écoulant dans l'hydrocyclone en un trajet circulaire présentant un rayon maximal de 1 à 50 cm; le quotient du carré de la vitesse d'écoulement, à l'entrée, du mélange liquide contenant des solides, d'une part, divisé par le rayon maximal du cercle parcouru dans l'hydrocyclone par un mélange liquide contenant des solides, d'autre part, se situant entre 50 et 2000 m/s$^2$, et le rapport entre la distance séparant l'entrée du mélange liquide contenant des solides à la partie supérieure de l'hydrocyclone et la sortie, à l'extrémité inférieure de l'hydrocyclone, du mélange liquide enrichi en des solides, d'une part, et le rayon maximal du cercle parcouru par le mélange liquide contenant des solides et circulant dans l'hydrocyclone, d'autre part, vaut de 3 à 30.

2. Procédé selon la revendication 1, caractérisé en ce que le mélange liquide enrichi en des solides et retiré de l'hydrocyclone est soumis à une sédimentation des particules solides, sans application de forces centrifuges.

3. Procédé selon la revendication 1 ou 2, caractérisé en ce qu'on sépare par évaporation au moins une partie des constituants liquides du mélange liquide, enrichi en des solides et qui est évacué de l'hydrocyclone.

4. Procédé selon une ou plusieurs des revendications 1 à 3, caractérisé en ce que le mélange liquide contenant des solides, contient, de façon prédominante, des hydrocarbures chlorés comportant 2 atomes de carbone.

5. Dispositif pour la mise en oeuvre du procédé selon la revendication 1, consistant en : un hydrocyclone (2) ayant un conduit (1) d'arrivée, un conduit d'évacuation à la partie supérieure (3) et un conduit d'évacuation à la partie inférieure (4), qui débouche dans un récipient (5) cylindrique vertical dont la partie inférieure se rétrécit coniquement et s'y termine au voisinage de l'axe du cylindre à 20 % au moins de la hauteur totale interne du récipient (5) au-dessous de la partie supérieure de ce récipient, un conduit (6) d'évacuation à la partie supérieure (5), qui rejoint le conduit (3) sortant de l'hydrocyclone (2), et un conduit (7) d'évacuation situé à la partie inférieure du récipient (5) et qui contient un organe (8) de fermeture ou d'obturation.

6. Dispositif selon la revendication 5 qui contient en outre un conduit (9) comportant un organe (10) de fermeture et une pompe (12), qui relie le conduit (6) d'évacuation au conduit (1) d'arrivée et qui bifurque du conduit (6) d'évacuation, entre le récipient (5) et la jonction avec le conduit (3) d'évacuation, ainsi qu'un organe (11) de fermeture, monté dans le conduit (6) d'évacuation, entre la bifurcation du conduit (9) et la jonction avec le conduit (3) d'évacuation.